# EUROPÄISCHE PATENTANMELDUNG

(11) **EP 4 111 866 A1**
(43) Veröffentlichungstag der Anmeldung: **04.01.2023**
(21) Anmeldenummer: 22182080.6
(22) Anmeldetag: 30.06.2022
(51) Int. Cl.: A22C 11/02, A22C 17/00, A23L 17/00, G01N 33/12

(54) **VERFAHREN ZUR STEUERUNG EINER VORRICHTUNG ZUM HERSTELLEN EINES LEBENSMITTELS**

(30) Priorität: 30.06.2021 DE 102021116879
(71) Anmelder: Inotec GmbH Maschinenentwicklung und Vertrieb, 72770 Reutlingen (DE)
(72) Erfinder: SCHWENK, Holger, 71111 Waldenbuch (DE)
(74) Vertreter: Patentanwälte und Rechtsanwalt Weiß, Arat & Partner mbB

(57) **Zusammenfassung**

Bei einem Verfahren zur Steuerung einer Vorrichtung (P) zum Herstellen eines Lebensmittels, insbesondere in der fleischverarbeitenden Industrie, bei der das Lebensmittel zumindest eine Bearbeitungsstation durchläuft und aus dieser aus einem Austrag (10) ausgetragen wird, sollen an dem Austrag (10) Parameter über das Lebensmittel ermittelt und zur Überwachung und/oder Änderung des Lebensmittels Prozess- und/oder Maschinenparameter in der Vorrichtung (P) verändert werden.

## Beschreibung

### Technisches Gebiet

Die Erfindung betrifft ein Verfahren zur Steuerung einer Vorrichtung zum Herstellen eines Lebensmittels, insbesondere in der fleischverarbeitenden Industrie, bei der das Lebensmittel zumindest eine Bearbeitungsstation durchläuft und aus dieser aus einem Austrag ausgetragen wird.

### Stand der Technik

Definitionsgemäss sind Lebensmittel Substanzen, die konsumiert werden, um den menschlichen Körper zu ernähren. Dazu gehören zum einen fertige Lebensmittel, welche die Natur dem Menschen anbietet und welche er in der angebotenen Zusammensetzung auch zu sich nimmt. Eine Vielzahl von Lebensmitteln bestehen aber aus Substanzen, die miteinander vermischt und/oder zerkleinert oder anderweitig behandelt werden müssen, bevor sie als Lebensmittel verzehrt werden. Gerade in der fleischverarbeitenden Industrie gibt es bereits fertige Fleischstücke, die lediglich zurechtgeschnitten und beispielsweise gewürzt werden müssen, aber auch eine Vielzahl von Produkte, bei denen das Fleisch gemischt und/oder zerkleinert wird. So zeigt z.B. die DE 199 60 409 A1 eine Vorrichtung zum Zerkleinern eines Zerkleinerungsgutes mit mehreren Lochplatten und davor rotierenden Schneidköpfen mit einstellbarem Spalt, wobei das Fleisch durch die Schneidköpfe zerkleinert und durch die Lochplatten gedrückt wird. Eine derartige Vorrichtung wird auch als Wolf bezeichnet.

Die DE 10 2017 123 164 zeigt einen Mischer mit einem grossen Trog, in dem sich Mischelemente um eine Welle bewegen und das entsprechende Fleischprodukt vermischen.

### Aufgabe der Erfindung

Aufgabe der vorliegenden Erfindung ist es, ein Verfahren der o.g. Art zu schaffen, bei dem die Qualität eines Produktes, insbesondere seine Zusammensetzung und Homogenität verbessert werden soll.

### Lösung der Aufgabe

Zur Lösung der Aufgabe führt, dass an dem Austrag Parameter über das Lebensmittel ermittelt und zur Überwachung und/oder Änderung des Lebensmittels Prozess- und/oder Maschinenparameter in der Vorrichtung verändert werden.

Das heisst, es soll nicht mehr dem Zufall überlassen werden, wie das Lebensmittel zusammengesetzt ist, sondern es soll gezielt überwacht und ggf. verändert werden. Das letztendliche Ziel wäre, dass die genauen Prozess- und Maschinenparameter ermittelt werden, mit denen ein Lebensmittel der festgestellten optimalsten Qualität erzeugt wird. Die dazu gehörenden entsprechenden Prozess- und Maschinenparameter werden dann gesammelt und können so auch z.B. anderen Maschinen zur Verfügung gestellt werden. Hierdurch wird gewährleistet, dass immer ein Lebensmittel von der gewünschten Qualität hergestellt wird. Sollte dieses Lebensmittel beispielsweise nach einer gewissen Dauer seiner Herstellung nicht mehr die gewünschte Qualität aufweisen, so ist aus den bisher durchgeführten Korrelationen zwischen Parametern des optimalen Lebensmittels und gewählten Prozess- bzw. Maschinenparametern ableitbar, welche Maschinen- und/oder Prozessparameter verstellt werden müssen, um die Qualität des Lebensmittels wieder zu erzielen. Erfindungsgemäss findet somit ein ständiger Austausch und Abgleich der Parameter des Lebensmittels und der Prozess- bzw. Maschinenparametern statt. Eine Änderung kann in Echtzeit stattfinden.

In einem bevorzugten Ausführungsbeispiel der Erfindung erfolgt die Ermittlung der Parameter des Lebensmittels und insbesondere von dessen Zusammensetzung und Homogenität durch eine Spektralanalyse. Hierzu ist auf dem Markt der sogenannte NIRONE-Sensor bekannt, mit dem aufgrund von Spektralanalyse der Wasser-, Fett-, Protein- und Salzgehalt eines Lebensmittels aus Fleisch ermittelt werden kann. Mit diesem NIRONE-Sensor wird ein Spektrum eines optimalen Lebensmittels, beispielsweise einer hergestellten Wurst, ermittelt, gleichzeitig werden eine Vielzahl von Prozess- bzw. Maschinenparameter mit Hilfe von geeigneten Sensoren an oder in der Maschine abgegriffen und in das Verhältnis zu den Parametern des Lebensmittels gesetzt.

Natürlich ist auch daran gedacht, in vorangehenden Verfahren zu ermitteln, wie sich das Lebensmittel bzw. welche Parameter des Lebensmittels sich bei Änderung bestimmter Prozess- bzw. Maschinenparametern verhält. Daraus ergibt sich die Grundlage für eine Änderung eines bestimmten Prozess- bzw. Maschinenparameters, falls sich ein bestimmter Parameter des Lebensmittels verändert. Auf diese Weise könnte eine ganze Datenbank für Soll-Parameter zur Erzielung von bestimmten optimalen Ausgestaltungen von Lebensmittel erzeugt werden. Dies alles kann über eine entsprechende Maschinensteuerung in Echtzeit durchgeführt werden.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine perspektivische und schematische Darstellung einer erfindungsgemässen Vorrichtung zur Herstellung eines Lebensmittels;
Figur 2 ein Diagramm einer erfindungsgemässen Spektralanalyse;
Figur 3 einen Längsschnitt durch einen Zerkleinerer;
Figur 4 ein Blockdiagramm des erfindungsgemässen Verfahrens.

### Ausführungsbeispiel

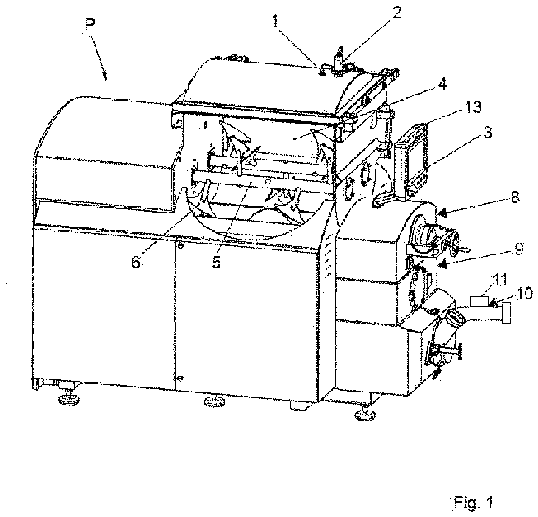

Als Teil einer erfindungsgemässen Vorrichtung zum Herstellen eines Lebensmittels ist in Figur 1 ein Mischer P mit geöffnetem Mischraum 4 zu erkennen. In diesem Mischraum 4 sind zwei Wellen 5.1 und 5.2 dargestellt, an denen sich Mischelemente 6 befinden, die der Vermischung eines Lebensmittels dienen.

Dem Mischraum 4 ist eine Beleuchtung 2 zugeordnet, mit der der Mischraum 4 ausgeleuchtet werden kann. Ebenso ist dem Mischraum 4 eine Videokamera 1 zugeordnet, über die Bilder des Mischraums 4 auf einen Bildschirm 3 übertragen werden können. Der Bildschirm 3 zeigt bevorzugt auch weitere Steuerungselemente für den Mischer P.

Von dem Mischraum 4 wird das vermischte Produkt weitergeleitet in einen Zerkleinerer 8, wobei z.B. im Falle der Verarbeitung von Fleisch dieser Zerkleinerer 8 ein Wolf ist. Mittels einer Pumpe 9 wird dann das Produkt aus einem Austrag 10 ausgetragen und beispielsweise in eine Abfülleinrichtung übergeführt. Dabei ist erfindungsgemäss dem Austrag 10 ein Sensor 11 aufgesetzt, der eine Vielzahl von Parametern des auszutragenden Produktes ermittelt.

Im Falle der Herstellung eines Fleischproduktes, insbesondere einer Wurst handelt es sich bei dem Sensor 11 um einen NIRONE-Sensor, welcher ein Spektralsensor ist, der das MIR-Spektrum im Wellenlängenbereich von 1550 nm bis 1950 nm misst. Ferner beinhaltet er bevorzugt auch zusätzlich einen RGB-Farbsensor. Der Spektralsensor basiert auf einem mikroelektro-mechanischen NIR-Bereich durchstimmbaren Fabry-Perot Interferometer und ist programmierbar. U.a. lässt sich festlegen, ob er den gesamten Wellenlängenbereich oder nur ausgewählte Wellenlängen scannt. Unter NIR wird das sogenannte Nahe Infrarot oder Nahinfrarot verstanden, das den Bereich des elektromagnetischen Spektrums bezeichnet, der sich in Richtung grösserer Wellenlänge an das sichtbare Licht anschliesst. Dabei handelt es sich um den Bereich von 780 nm bis 1400 nm. Er umfasst somit die Spektralbereiche IR-A und IR-B.

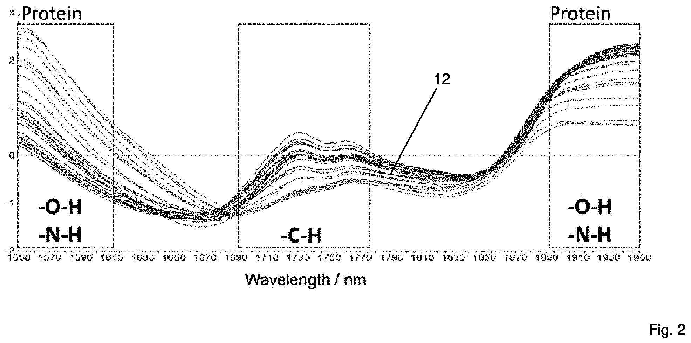

In Figur 2 ist das Ergebnis einer Spektralanalyse von einem Fleischprodukt hergestellt, welches durch die Spektralanalyse auf Wasser und Protein im Wellenlängenbereich von 1550 bis 1610, auf Fett im Längenbereich von 1690 bis 1770 und nochmals auf Wasser und Protein im Wellenlängenbereich von 1890 bis 1950 untersucht wird. Die so ermittelten Kurven 12 hängen nun von sehr vielen anderen Parametern der erfindungsgemässen Vorrichtung ab bzw. werden durch diese Parameter bestimmt. Hierzu zählen beim Mischer vor allem
- die Anzahl der Mischelemente
- das Drehmoment der Mischwellen, auf denen die Mischelemente befestigt sind
- die Drehzahl der Wellen 5
- die Temperatur im Mischraum u.a.

Beim Zerkleinerer massgeblich sind
- die Geschwindigkeit, mit der die Elemente (Messerplatte über Lochplatte) drehen
- das Drehmoment der Wellen, an denen die Zerkleinerungselemente angeordnet sind
- ein Druck, der in dem Zerkleinerungsraum herrscht
- die Temperatur im Zerkleinerungsraum
- der Druck, mit dem sich einzelne Elemente im Zerkleinerungsraum gegeneinander abstützen, vor allem die Messerplatte gegen die Lochplatte u.a.

Für die Ermittlung dieser Parameter, zumindest zum Teil, sollen entsprechende Sensoren angeordnet sein, die sowohl Prozessparameter als auch Maschinenparameter ermitteln. Diese werden in einem entsprechenden Prozessor 13 gesammelt, der hinter dem Bildschirm 3 angeordnet ist.

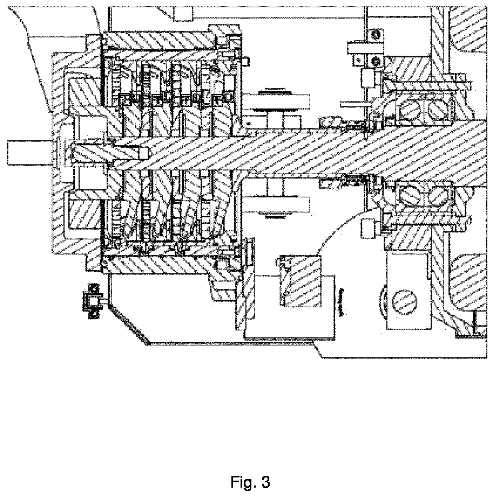

Nur als Beispiel ist in Figur 3 ein Längsschnitt durch einen entsprechenden Zerkleinerer angedeutet, in welchem Drucksensoren, Thermoelemente und Piezoelemente integriert sind.

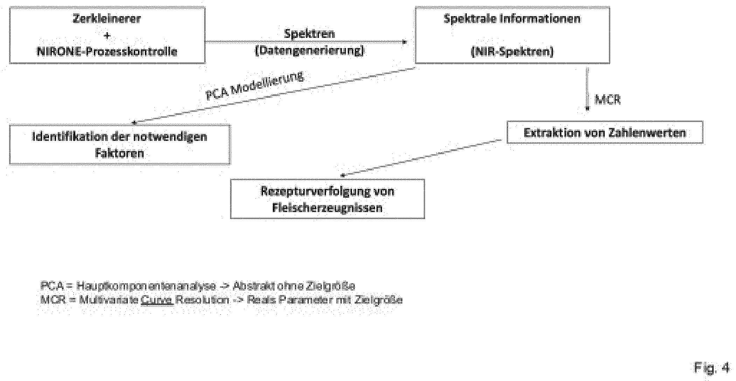

In Figur 4 ist als Ausführungsbeispiel eines erfindungsgemässen Verfahrens die Überwachung eines Zerkleinerers angedeutet. Dieser Zerkleinerer unterliegt, wie auch in Figur 1 gezeigt, einer NIRONE-Prozesskontrolle, d.h., am Austrag 10 befindet sich der Sensor 11. Dieser Sensor generiert Spektren des Produktes, welches den Zerkleinerer verlässt, wobei die entsprechenden Daten dem Prozessor 13 zugeführt werden. Dieser beinhaltet dann die spektralen Informationen bzw. NIR-Spektren. Die Spektren zeigen eine gewisse Zusammensetzung des Produktes insbesondere im Hinblick auf den Wasser-, Fett-, Protein- und Salzgehalt. Durch das Übereinanderlegen von aufeinanderfolgenden Spektren kann auch eine Homogenität des Produktes in der Länge ermittelt werden.

Nunmehr erfolgt eine Identifikation der notwendigen Faktoren, welche zu der ermittelten Zusammensetzung des Produktes führen bzw. diese bestimmen. Es wird z.B. ermittelt, wie die Produktzusammensetzung durch Veränderung der Temperatur oder des Druckes in dem Zerkleinerer bzw. zwischen den Zerkleinerungselementen beeinflusst wird.

Danach erfolgt eine Extraktion von Zahlenwerten, die letztendlich zum Aufbau einer Datenbank von Rezepturen führen. In der Datenbank für diese Maschine bzw. für diesen Maschinenbestandteil wird niedergelegt, beispielsweise welcher Aufbau bzw. welche Homogenität das Produkt bei welchen eingestellten Parametern hat. Diese Werte können jederzeit als Referenzkurven dienen und auch auf andere Vorrichtungen bzw. Vorrichtungsteile übertragen werden.

Obwohl nur einige bevorzugte Ausführungsbeispiel/e der Erfindung beschrieben und dargestellt wurden, ist es offensichtlich, dass der Fachmann zahlreiche Modifikationen hinzufügen kann, ohne Wesen und Umfang der Erfindung zu verlassen.

**Bezugszeichenliste**

| | | | | | |
|---|---|---|---|---|---|
| 1 | Videokamera | 34 | | 67 | |
| 2 | Beleuchtung | 35 | | 68 | |
| 3 | Bildschirm | 36 | | 69 | |
| 4 | Mischraum | 37 | | 70 | |
| 5 | Welle | 38 | | 71 | |
| 6 | Mischelement | 39 | | 72 | |
| 7 | | 40 | | 73 | |
| 8 | Zerkleinerer | 41 | | 74 | |
| 9 | Pumpe | 42 | | 75 | |
| 10 | Austrag | 43 | | 76 | |
| 11 | Sensor | 44 | | 77 | |
| 12 | Kurven | 45 | | 78 | |
| 13 | Prozessor | 46 | | 79 | |
| 14 | | 47 | | | |
| 15 | | 48 | | | |
| 16 | | 49 | | | |
| 17 | | 50 | | P | Vorrichtung |
| 18 | | 51 | | | |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Verfahren zur Steuerung einer Vorrichtung (P) zum Herstellen eines Lebensmittels, insbesondere in der fleischverarbeitenden Industrie, bei der das Lebensmittel zumindest eine Bearbeitungsstation durchläuft und aus dieser aus einem Austrag (10) ausgetragen wird,
**dadurch gekennzeichnet,**
**dass** an dem Austrag (10) Parameter über das Lebensmittel ermittelt und zur Überwachung und/oder Änderung des Lebensmittels Prozess- und/oder Maschinenparameter in der Vorrichtung (P) verändert werden.

2. Verfahren nach Anspruch 1, **dadurch gekennzeichnet, dass** die am Austrag (10) ermittelten Parameter den ebenfalls ermittelten Prozess - und/oder Maschinenparametern zugeordnet werden.

3. Verfahren nach Anspruch 2, **dadurch gekennzeichnet, dass** aus der Zuordnung der am Austrag (10) ermittelten Parametern zu den ebenfalls ermittelten Prozess - und/oder Maschinenparametern Rezepturen für das Lebensmittel hinterlegt werden.

4. Verfahren nach wenigstens einem der vorhergehenden Ansprüche, **dadurch gekennzeichnet, dass** am Austrag (10) zumindest die Zusammensetzung des Lebensmittels ermittelt wird.

5. Verfahren nach wenigstens einem der vorhergehenden Ansprüche, **dadurch gekennzeichnet, dass** am Austrag (10) die Homogenität des Lebensmittels ermittelt wird.

6. Verfahren nach wenigstens einem der vorhergehenden Ansprüche, **dadurch gekennzeichnet, dass** am Austrag (10) bei einem Lebensmittel der fleischverarbeitenden Industrie zumindest einer der Gehalte an Wasser, Fett, Protein oder Salz ermittelt wird.

7. Verfahren nach wenigstens einem der vorhergehenden Ansprüche, **dadurch gekennzeichnet, dass** als Maschinenparameter insbesondere eine Geschwindigkeit, ein Drehmoment von Drehelementen und/oder ein Druck von zusammenwirkenden Elementen und/oder eine Temperatur von Arbeitselementen ermittelt wird.

8. Verfahren nach wenigstens einem der vorhergehenden Ansprüche, **dadurch gekennzeichnet, dass** als Prozessparameter insbesondere eine Temperatur und/oder ein Druck in einem Prozessraum (4) ermittelt wird.

9. Verfahren nach wenigstens einem der vorhergehenden Ansprüche, **dadurch gekennzeichnet, dass** am Austrag (10) eine Spektralanalyse des Lebensmittels erfolgt.
